# EUROPEAN PATENT SPECIFICATION

(11) **EP 2 895 137 B1**
(45) Date of publication and mention of the grant of the patent: **09.11.2016**
(21) Application number: 13773408.3
(22) Date of filing: 02.09.2013
(51) Int. Cl.: A61J 1/20, A61M 5/315, A61M 5/20, B65B 3/00, G06F 19/00

(54) **APPLIANCE FOR THE TRANSFER OF BIOMEDICAL FLUIDS BETWEEN HOSPITAL RECEPTACLES**
VORRICHTUNG ZUR ÜBERTRAGUNG VON BIOMEDIZINISCHEN FLÜSSIGKEITEN ZWISCHEN KLINIKBEHÄLTERN
APPAREIL DE TRANSFERT DE FLUIDES BIOMÉDICAUX ENTRE DES RÉCEPTACLES D'HÔPITAL

(30) Priority: 17.09.2012 IT MO20120219
(43) Date of publication of application: 22.07.2015
(73) Proprietor: Pierc di Giovanelli Gabriele E C. S.A.S, 41032 Cavezzo (MO) (IT)
(72) Inventor: MAFFEI, Giuseppe, 41037 Mirandola (IT)
(74) Representative: Brunacci, Marco
(86) International application number: PCT/IB2013/058226
(87) International publication number: WO 2014/041461

(56) References cited:
- EP-A1- 2 335 755
- WO-A1-2012/063111
- US-A- 5 911 252

## Description

### Technical Field

The present invention relates to an appliance for the transfer of biomedical fluids between hospital receptacles.

### Background Art

In detail, the invention relates to an appliance to take, transport and dose biomedical fluids.

Different types of therapeutic treatment, such as cancer treatment, require patients to be connected to an infusion line for the parenteral administration of drugs (e.g., a phleboclysis), such line being placed in communication with a series of bags containing the doses of drugs to be administered.

Each bag is connected to the infusion line, by means of an inlet tube, in a way independent of the others, thanks to the use of a suitable series of valves, and the dispensing of drugs to the patient occurs in the succession provided by the therapy, placing between the administering of one drug and the next an intermediate phase of cleaning of the infusion line by means of a saline solution wash, so as to prevent any type of contamination between the drugs.

The administering of the drugs continues until the complete emptying of the bags containing the respective doses of drugs prescribed by the doctor, thus carrying out the correct therapeutic treatment.

The introduction of the drug into the respective bags is done by a hospital operator who doses the necessary quantity inside the bags themselves.

The drugs to be introduced into the bags of the infusion line are kept in receptacles provided for the purpose, such as bottles or further bags, normally located in the hospital pharmacy, and are from time to time taken by the above personnel using specific instruments or appliances.

In particular, the patent nr. WO2012063111, in the name of the Applicant of the present patent application, covers an appliance for the transfer of biomedical fluid, usable precisely to take doses of liquid drug from the receptacles wherein they are temporarily stored and to introduce such doses into the bags of the infusion line.

Such appliance first of all comprises a containment syringe body which includes a sliding piston inserted sealed in said syringe body.

Furthermore, the appliance comprises a grip having a coupling seat to attach to it the syringe body in a removable way, which grip includes motor means to operate a thrust rod suitable for coupling to the above piston of the syringe body, and further includes control means of the motor means for the volumetric dosing of the biomedical fluid.

In practice, at one end of the syringe body a cap is fastened which shapes a Luer-lock connector, or similar association means with the openings of the receptacles and, at the opposite end, the syringe body comprises coupling means designed to fasten it to the grip, the syringe body, the piston and the cap in practice making up a replaceable cartridge, which is used in the following manner.

The operator attaches such cartridge to the grip by means of the respective coupling means.

Subsequently, by means of the Luer-lock connector, the operator connects the cartridge to the opening of a bag filled with biomedical fluid, so as to put the respective internal volumes in communication, after which, by means of the control means, which control the movement of the piston by means of the thrust rod, he/she takes the suitable dose (or several doses) of fluid from the bag, so this flows into the syringe body.

At this point, the operator takes the dose of fluid to the infusion line and, by means of the control means, injects into a given bag of the line exactly the quantity of fluid making up the dose envisaged for such bag, according to the pre-established therapy.

This operation is repeated for each drug dose transfer, being careful to replace the cartridge used to transfer a given biomedical fluid, before taking a different fluid, so as to avoid any contamination which could be very hazardous for the patients' health.

If more transfers have to be made of the same biomedical fluid, the same cartridge can be used, albeit only for a limited number of times.

The appliance described above has represented a significant step forward for the sector in terms of technology, nevertheless the applicant has provided a number of upgrades that could result in considerable benefits and overcome the problems referred to below.

As said, the cartridge is not of the disposable type and its re-use is therefore envisaged.

Nevertheless, this exposes the transfer operation to a possible risk: the appointed health operator could make the mistake of using a cartridge that once contained a given drug to transfer a completely different drug, thereby contaminating the latter, and this could also result in very negative consequences for the health of the patient connected to the infusion line, if the two drugs that come into contact interact in a harmful way.

Furthermore, the operator who intends using a given cartridge could also not precisely know for how long this has been used and this is particularly important because laboratory tests have shown that, with the passing of time, the inner surface of the syringe body (normally made of polycarbonate or polyethylene or similar plastic materials), and the surface of the piston can release substances that represent a contaminant for the biomedical fluids.

In practice, the health operator finds him/herself in the uncertainty of whether or not a given cartridge has terminated its cycle of use.

This circumstance can lead both to the risk of contamination described above and, conversely, to an excessively-limited re-use of the cartridge, in the event of the operator, because of the mentioned uncertainty, deciding for safety's sake to in any case dispose of the used cartridge independently of the time that has elapsed since it was first used.

Furthermore, both for economic reasons and for safety reasons, the need, thus far unsatisfied, has arisen to know exactly, for each biomedical fluid, the quantity used during each single therapeutic session and exactly when doses of it were taken from the pharmacy for use in the infusion lines.

In fact, such information would permit not only better planning as regards the purchase of the biomedical fluids used in clinics by the relative pharmacy, but also the real-time monitoring of their relative stock, as well as also making it possible to check that no thefts have occurred and keep a trace of the use of the drugs in the specific therapeutic prescriptions, so as to be able to promptly intervene in case of errors, i.e., in the event of a health operator making a mistake as regards the doses or even the type of biomedical fluid administered to a patient.

### Description of the Invention

The main aim of the present invention is to provide an appliance for the transfer of biomedical fluids which prevents the risk of the cartridge itself being used by mistake to transfer two different fluids.

Another object of the invention is to provide a transfer appliance which permits knowing for how long a given cartridge has been used to transfer a biomedical fluid.

A further object of the invention is to provide an appliance for the transfer of biomedical fluids which permits the health operator or the pharmacy staff of a clinic to know exactly, for each biomedical fluid and each cartridge, the quantity of fluid transferred both during the individual transfers and within a given period of time, and when the individual transfers were made of the various biomedical fluids and other information still.

Yet another object of the present invention is to make available a syringe device designed so as to completely prevent contaminations of the introduced liquid by elements in the air, during the period of non-use between two subsequent transfers.

Another object of the present invention is to provide an appliance for the transfer of biomedical fluids which allows to overcome the mentioned drawbacks of the state of the art within the ambit of a simple and effective to use as well as low cost solution.

The above objects are achieved by the present appliance for the transfer of biomedical fluids between hospital receptacles, suitable for taking, transporting and dispensing doses of biomedical fluid, according to claim 1.

### Brief Description of the Drawings

Other characteristics and advantages of the present invention will become more evident from the description of a preferred, but not sole, embodiment of an appliance for the transfer of biomedical fluids, illustrated purely as an example but not limited to the annexed drawings in which:
Figure 1 is a schematic view of the storage receptacles of the biomedical fluids, of an above mentioned infusion line, and of several appliances according to the invention used in the transfer of fluid from the first to the second ones;
Figure 2 is an axonometric view of the syringe device of the appliance according to the invention;
Figure 3 is an exploded view of the appliance according to the invention; and
Figure 4 is a lateral view, partly in vertical section along the A-A half-plane and, for the remaining part, partially broken-out, with a schematic representation inside the operating device.

### Embodiments of the Invention

With particular reference to such figures, indicated by 1 is the appliance for the transfer of biomedical fluids between hospital receptacles according to the invention.

In detail, the proposed appliance 1 is suitable for taking, transporting and dispensing doses of biomedical fluid.

Notice how, in the present description, by the term "biomedical fluid" is meant any fluid used in the medical/hospital field and intended to be administered to a patient by means of bags or other receptacles and which requires precise pre-dosing.

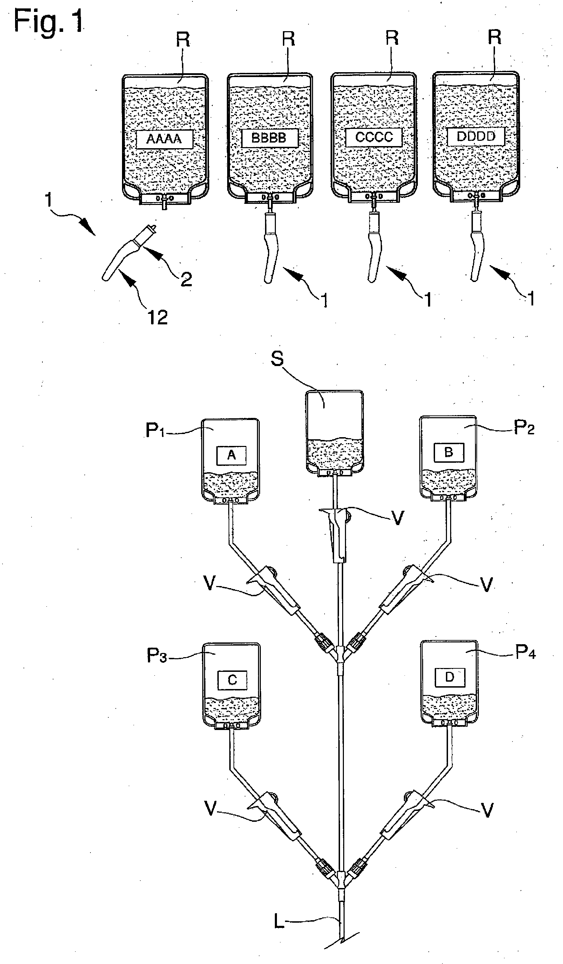

In the example shown in figure 1, the biomedical fluids consist of a series of drugs, like those in use for cancer therapies, contained in respective first receptacles R, which in the case in question are hospital bags (but the invention can be used with other types of receptacles such as bottles or the like).

As already said during discussion of the prior art, from the first bags R the biomedical fluids must be transferred into the second receptacles P, in particular bags or the like, to be connected to an infusion line L having a bag S of saline solution and having specific cut-off valve means V.

The proposed appliance 1 has been designed for such transfer.

The known aspects of the transfer are not repeated here for the sake of brevity; suffice it to say that the invention can be used to transfer biomedical fluids with the same applications already described in the aforementioned patent application nr. WO2012063111 which, therefore, in relation to these aspects, shall be deemed integrated here for reference.

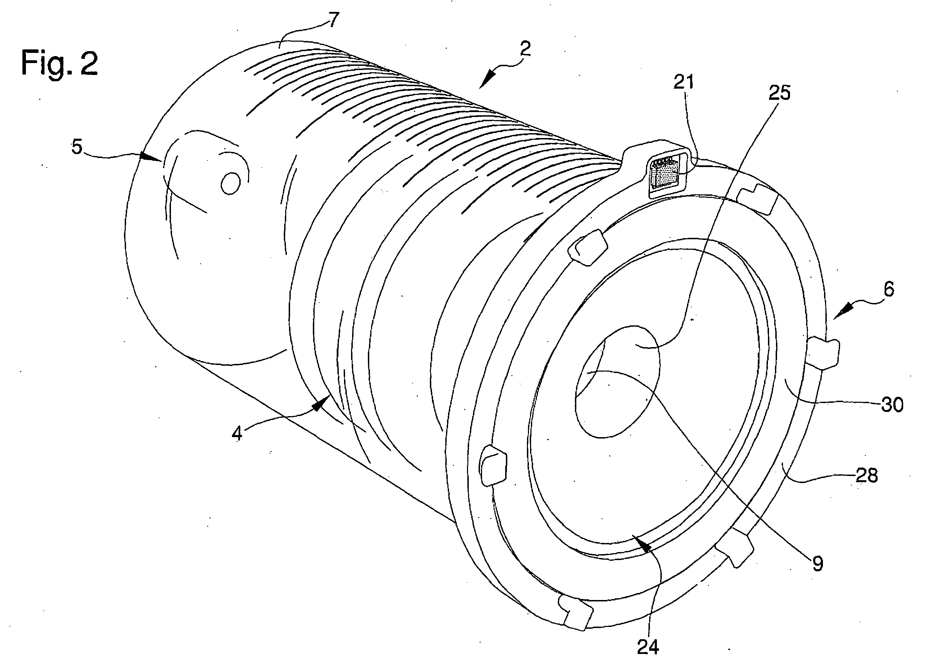

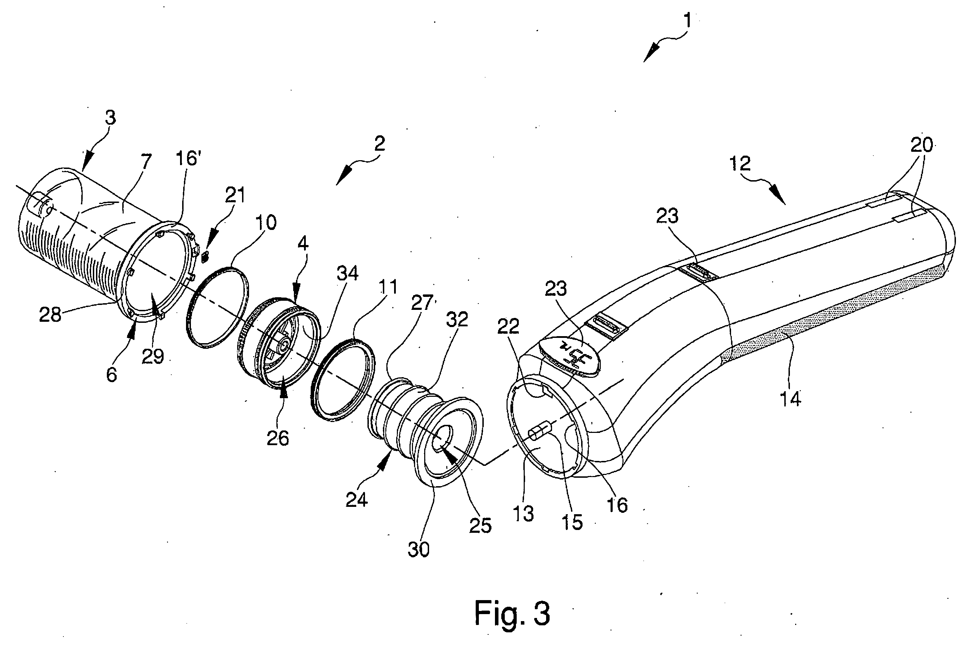

The appliance 1 first of all comprises a syringe device 2 (shown in figure 2) in turn comprising a hollow syringe body 3 for containing said biomedical fluid, and also comprising at least one sliding piston 4 fitted sealed in said syringe body 3 (see also figure 3).

The syringe body 3, in practice a hollow body with a function similar to the so-called "small barrels" of traditional syringes, comprises a first end 5, having joining means to put in communication its inside with that of a hospital receptacle R, P (e.g., consisting of a Luer-lock connector), and a second open end 6 (see in particular the figure 2).

Preferably, the syringe body 3 is tubular, with a cylinder-shaped side wall 7 and can be made of plastic material (polyurethane, polycarbonate, polyethylene, etc...), e.g. transparent, in which case a graduated scale can be shown, or it can be colored, opaque, etc....

The dimensions of the syringe body 3 can vary according to the required containment capacity (e.g.: 10 ml, 20 ml, 30 ml, 50 ml or 60 ml).

The piston 4 defines inside the syringe body 3 two variable-volume chambers 8, 9, seal-separated by the piston 4 itself, the chambers 8, 9 varying their dimensions according to the position of the piston 4 inside the syringe body 3, i.e., its arrangement along the longitudinal extension of the syringe body 3 itself.

The piston 4 preferably has a substantially cylinder shape with the side surface in contact with the inner surface of the side walls of the syringe body 3; furthermore, to the longitudinal ends of the piston 4 seal means 10, 11, such as O-rings or the like, can be fastened or firmly coupled (see figure 3).

As will be better explained below, the syringe device 2 in practice represents an interchangeable cartridge designed to transfer a given biomedical fluid.

The proposed appliance 1 further comprises an operating device 12 that can be gripped (see figure 3), which in turn comprises a fastening seat 13 which can be coupled in a removable way to said second end 6 of the syringe device 2 (by means of releasable coupling means 16, 16', such as bayonet couplings or the like).

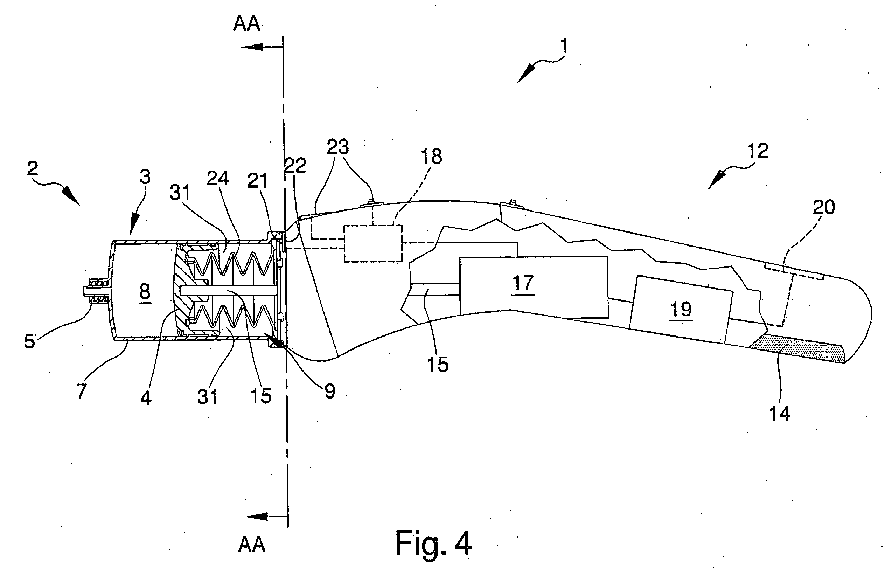

The operating device 12 can have a casing shaped like a grip (as shown in the figures 3 and 4), an ergonomic shape and, e.g., a portion of anti-slip material 14 to help gripping by the user.

As shown in the figures 3 and 4, the operating device 12 first of all includes a thrust rod 15 suitable for sliding longitudinally through said second end 6, following the coupling between said syringe device 2 and said operating device 12.

In practice, the syringe device 2 is meant to be fitted on the operating device 12 in its fastening seat 13, preferably placed at one front end, at which seat, the thrust rod 15 exits from the rest of the operating device 12 and is meant to cross the second end 6 of the syringe device 2 so as to be able to reach the piston 4.

Temporary fastening means (such as a bayonet coupling or other coupling means suitable for the purpose) are therefore provided for the releasable coupling between the rod and the piston 4, suitable for enabling the operation of the rod to produce the sliding of the piston 4 within the syringe body 3 (and therefore the taking or dosing of the fluid).

For the purpose of operating the rod 15, the operating device 12 includes motor means 17 connected to the thrust rod 15, and furthermore, it includes control means 18 (only schematically shown in the figure 4) connected to said motor means 17 so as to be able to control the volumetric dosage of the quantity of fluid taken from one of the first hospital receptacles R and the volumetric (and also micro-volumetric) dosage of the quantity of fluid dispensed in one of the second hospital receptacles P.

The motor means 17 can include one or more actuators, including e.g. a linear actuator for controlling the longitudinal sliding movement of the thrust rod 15.

Furthermore, the motor means 17 can also be connected to a power supply battery 19, e.g. rechargeable, fitted in the operating device 12, which can be associated with lead connections 20 for connecting to a recharge station.

In an innovative and original way, the appliance 1 of the invention comprises storage means 21 (such as EEPROM memories or other electronic storage devices such as RFID tag, etc..), mounted on the syringe device 2 (and which can be seen in the figures 2 and 3), and able to store information relating to the type of transferred fluid, the quantity of taken fluid, the quantity of dispensed fluid, when each dose has been taken and/or dispensed and how many doses have been dispensed/taken (and if necessary also other information, as will be explained later on).

For the purpose of cooperating with such storage means 21, the operating device 12 advantageously comprises reading/writing means 22 for reading and writing the information from/on the storage means 21 of the syringe device 2, and furthermore, the above control means comprise a processing unit 18 able to control the reading/writing means 22 and also able to process said information according to a predefined program, the processing unit 18 can be connected to external electronic devices (by means of electronic Wi-Fi connection or by means of USB ports, etc...).

Preferably, the storage means 21 are mounted on the second end 6 of the syringe device 2 and the reading/writing means 22 are positioned at the fastening seat 13 of the operating device 12.

In this case, the storage means 21 and the reading/writing means 22 are arranged in such a way that, upon the coupling between the syringe device 2 and the operating device 12, they are directly connected the ones to the others.

In practice, the aforementioned coupling means between the two devices of the appliance 1, both when they are either of the bayonet type or means of different type, are configured in such a way that, when the syringe device 2 is correctly fitted on the operating device 12, then the two devices are in such a position the one to the other that the storage means 21 are directly in contact with the reading/writing means.

The above control means 18 can also comprise a control interface 23 (which includes, for example, a keypad, a screen and other similar elements), mounted on the operating device 12, and connected to the processing unit 18 to enable the user to set the quantity of liquid of the doses and collect the information processed by it; furthermore, following the coupling between the syringe device 2 and the operating device 12, the control interface 23 also allows the user to control the dispensing and/or the taking of fluid, and furthermore to collect/transfer information from/onto the storage means 21 of the cartridge 2. Before illustrating further construction and functional aspects of the proposed appliance 1 in detail, it is explained below how the invention can be used in order to fully bridge the gap affecting prior art.

When the health operator attaches a cartridge (i.e., the syringe device 2) to the operating device 12 for the first time, he/she connects the first end 5 of the cartridge 2 to a first receptacle containing a fluid to be taken and commands the taking of the desired dose, then the above-mentioned processing unit 18 commands the reading/writing means 22 to record on the storage means 21 of the cartridge 2 the moment the cartridge 2 was used for the first time.

Furthermore, by means e.g. of a computer connected in the ways described above to the processing unit 18 of the operating device 12, the operator can record on the storage means 21 which specific biomedical fluid the cartridge 2 is meant to transfer.

Yet again, for each taking operation from a first receptacle and for each dispensing operation into a second receptacle, the processing unit 18 also commands the writing on the storage means 21 of the quantity of dose (or doses) taken or dispensed, and also records when the doses have been taken or dispensed.

Consequently, on the storage means 21 of the cartridge 2 in question, are contained all the information, trace of which cannot be kept by prior art, and this information can be transferred onto external devices by the processing unit 18.

The invention thus permits knowing which type of fluid has been previously transferred with that specific cartridge 2 and, consequently, in the event of this being reused, e.g., by another health operator also using another operating device 12 (naturally as long as this has been made in agreement with the invention), the reading/writing means 22 inform the processing unit 18, and therefore the operator, about which fluid is univocally intended for the cartridge 2, and this prevents the aforementioned risk of contamination between different fluids, explained during the discussion on prior art.

Furthermore, considering that in the information means, the information is recorded relating to the time that has elapsed since initial use, the processing unit 18 can be programmed to give a warning signal whenever a cartridge 2 is fitted on an activation device that has already been used for a period of time close to that in which the above release of contaminating substances can start to occur by the material used to make the syringe body 3 and the piston 4, spoken of during the discussion on the limits of prior art.

Consequently, a given syringe device 2 (or cartridge) will be used numerous times, in perfect safety, thus providing considerable operating efficiency and a relative economic saving, but will be disposed of before there is any chance of its deteriorating and releasing contaminating substances into the transferred pharmaceutical products.

Conversely, the activation devices can be reused for an endless number of times, being subject only to the usual wear affecting electromechanical devices, and consequently different cartridges can be fitted, also in the case of some of them having been used to transfer a fluid different to that of the others.

Furthermore, each cartridge 2 bears information about the quantity and the number of the dispensed doses and about the time these were dispensed, and therefore allows the hospital pharmacy to set up a database which organizes and manages all such information, permitting perfect planning as regards the purchase of the biomedical fluids, the monitoring (including in real time) of the relative stock, and checking the fact that thefts could have occurred or the fact that, for the specific patient therapies, the prescribed pharmaceutical products have in fact actually been used, in the right quantities, and that no mistakes have been made.

Preferably, the storage means 21 include pre-stored information able to identify the syringe device 2 onto which they are fitted.

Such identification information can be used to allow the operating device 12 to check whether the syringe device 2 used has not been modified and is only the one foreseen for functionally cooperating with the operating device 12 of the invention.

Always preferably, the aforementioned control means 18 are programmable so as to automatically set the doses of fluid to take and dispense, both in the case relating to doses of different medicinal substances to use in the same therapy or, on the contrary, in the case of doses of the same medicinal substance to dispense in bags included in different infusion lines and then connected to different patients, etc....

This way, the hospital operator does not have to be concerned about manually setting the quantity of fluid of each dose every time he/she has to transfer the fluid from the respective first bag R in which it is stored to a bag P included in an infusion line L.

At practical level, once the syringe device 2 and the operating device 12 have been coupled together, the processing unit 18 automatically commands the forward movement of the thrust rod 15 until this has coupled with the piston 4. In this phase, the processing unit 18 proceeds to identify the cartridge 2 by means of the reading/writing means 22 which connect up to the storage means 21 and, in the event of its recognizing the cartridge 2 as being authentic, it permits the subsequent operation of the motor means 17.

Then, from time to time, the processing unit 18 automatically drives the thrust rod 15, according to the predefined program; so this slides along stroke lengths corresponding to the quantity of biomedical fluid to be taken and/or dispensed.

The operator can nevertheless exploit the possibility of a transparent syringe body 3 with graduated scale (like that shown on the attached tables) in order to make a visual check of the taking or dispensing of the liquid, for the purpose of a definitive safety control.

A description is provided below of the structure and operation of the preferred embodiment of the invention.

As previously already partially said, in each cartridge 2, the piston 4 defines, inside the syringe body 3, separating them, a containment chamber 8, which ends up in the above first end 5, and a sliding chamber 9, ready to house said thrust rod 15 and ending up in said second end 6.

It stands to reason that such chambers 8, 9 have a variable volume, because the respective dimension depends on the position which the piston 4 has within the longitudinal extension of the cavity of the syringe body 3, such position being determined by the sliding of the thrust rod 15, once this has been coupled to the piston 4 itself.

Although obvious, it must be explained that when the appliance 1 is connected to a first receptacle R by means of the fastening means of the first end 5 of the syringe body 3, then when the piston 4 is operated by pulling it towards the second end 6 of the syringe body 3, the biomedical fluid is taken from the receptacle R and moved into said containment chamber 8, where it can be kept during the transport to the second receptacle P.

Conversely, clearly, when the first end 5 is connected to the second receptacle P and the piston 4 is pushed towards this, then the liquid is dispensed, or injected, into the second receptacle P and expelled from the containment chamber 8.

As explained, all these operations are managed by the already-described control means 18.

Having said this, the invention preferably and advantageously comprises a covering element 24 (see in detail the figures 3 and 4, but also the figure 2) arranged in said sliding chamber 9 and comprising an opening hole 25 which can be crossed by the thrust rod 15, to allow it to be temporarily fastened to the piston 4.

The covering element 24, in general terms, is configured in such a way as to cover the inner surface of the side wall of the sliding chamber 9, independently of the position of the piston 4 in said syringe body 3, so as to avoid that, upon the uncoupling of the syringe device 2 from the operating device 12, the air entering the syringe body 3 from its second end 6 can touch said inner surface.

Before detailing the preferred construction versions of the covering element 24 the advantages will be explained of its adoption in the invention.

Since, when the cartridge 2 is used for taking fluid, the biomedical fluid which has entered it touches the inner surface of the syringe body 3, then, despite the piston 4 being associated sealed with the syringe body 3 itself, it is in any case possible for an albeit very small quantity of fluid to remain attached to the inner surface of the syringe body 3 even after dispensing, i.e., even after the piston 4 has been made to slide right up to the first end 5, and has therefore carried the fluid out.

In practice, after dispensing, a small quantity of fluid can remain in the above sliding chamber 9, behind the piston 4, attached to the inner surface of the side wall 7 of the syringe body 3.

This quantity is very small because, during dispensing, the piston 4 which slides inside the syringe body 3 brushes the liquid in contact with the inner surface of the side walls of this, like a windscreen wiper, including thanks to the above-mentioned seal means 10, 11, which are fastened to the piston 4 itself.

After the cartridge 2 has been separated from the operating device 12, considering that the second end 6 is open, air can enter into the syringe body 3, which brings dust and other corpuscles with it, or substances dispersed in it, which, if the covering element 24 were not advantageously provided, could attach themselves to the liquid still in the sliding chamber 9, with the risk, during subsequent use, of the taken fluid being contaminated by it.

In practice, what happens is that, if a cartridge 2 is reused during taking, the piston 4 backs up and the inner surface of the syringe body 3 which was included in the sliding chamber 9, and which contains traces of previously transferred liquid (which could include contaminants if the covering element 24 were not present), therefore becomes the inner surface of the containment chamber 8, which comprises the "new" taken liquid, which therefore comes into contact with the aforementioned traces of previously transferred liquid.

It is therefore clear that the use of a covering element 24 like that of the invention completely prevents any possibility of contaminants reaching the containment (and dispensing) chamber 8 and, therefore, the cartridge 2 can be used several times, in a perfectly safe way, without any air contaminants having the least chance of reaching the infusion lines L.

According to a preferred construction aspect of the covering element 24, it is longitudinal shaped and made of an elastic and resilient material.

In this case, as can be seen in figure 3, the piston 4 comprises an abutment side 26, facing said sliding chamber 9, which abuts a first longitudinal end of the covering element 24, while the aforementioned second end 6 of the syringe body 3 comprises an abutment transversal frame 28 defined around a through hole 29, so the second longitudinal end 30 of the covering element 24 abuts precisely said abutment frame (see figures 2 and 3).

Usefully, the covering element 24 is inserted longitudinally pre-compressed in the sliding chamber 9 so that its first and second ends 27, 30 adhere to said abutment side 26 of the piston 4 and to said second end 6 of the syringe body 3 respectively, independently of the position of the piston 4, to define, between the outer side surface of the covering element 24 and said inner side surface of the syringe body 3, a sealed gap 31.

In practice, into the sealed gap 31 thus defined, air does not enter from the second opening of the syringe body 3 and, consequently, the complete certainty is obtained that the aforementioned possible contamination is totally avoided.

Preferably, as shown in the figures 3 and 4, the covering element 24 is tubular, axial-symmetric and comprises a side wall 32 shaped like a bellows so as to favor the variations of its longitudinal dimensions.

In order to even further ensure the protection of the inner surface of the containment chamber 8, it can be provided that the abutment side 26 of the piston 4 shapes an abutment seat 34, clearly visible in the figure 3, able to receive a portion of said covering element 24 which comprises said first end 27.

In practice, in this case, the piston 4 has a glass shape, with the closed bottom turned towards the first end 5 of the syringe body 3, and the opposite side 26 which shapes a concavity, defining the mentioned seat 34, at the centre of which the temporary fastening means are present for coupling the thrust rod 15 in a releasable way, in the way explained above.

It has in practice been ascertained how the described invention achieves the proposed objects, making available an appliance 1 for the transfer of biomedical fluids between hospital receptacles that fully overcomes the drawbacks of prior art.

## Claims

1. Appliance (1) for the transfer of biomedical fluids between hospital receptacles (R, P), suitable for taking, transporting and dispensing doses of biomedical fluid, comprising:
- at least a syringe device (2) in turn comprising a hollow syringe body (3) for containing said biomedical fluid, and also comprising at least one sliding piston (4) fitted sealed in said syringe body (3), the latter comprising a first end (5), which has joining means for putting in communication its inside with that of a hospital receptacle (R, P), and a second open end (6);
- at least a grippable operating device (12), comprising a fastening seat (13) which can be coupled in a removable way to said second end (6) of the syringe device (2), the operating device (12) comprising a thrust rod (15) suitable for sliding longitudinally through said second end (6), following the coupling between said syringe device (2) and said operating device (12);
- temporary fastening means for coupling said rod (15) to said piston (4) in a releasable way, able to allow that the operation of said rod (15) produces the sliding of said piston (4) in said syringe body (3);
- motor means (17) for operating said thrust rod (15), included in said operating device (12);
- control means (18), included in said operating device (12), and connected to said motor means (17) so as to control both the volumetric dosage of the quantity of fluid taken from a first hospital receptacle (R) and the volumetric dosage of the quantity of fluid dispensed in a second hospital receptacle (P);
said appliance (1) being **characterized by** the fact that:
- storage means (21) are mounted on said syringe device (2) for storing information relative to the type of transferred fluid, the quantity of taken fluid, the quantity of dispensed fluid, when each dose has been taken and/or dispensed and how many doses have been dispensed/taken;
- said operating device (12) comprises reading/writing means (22) for reading and writing said information from/on said storage means (21); and that
- said control means comprise a processing unit (18) able to control said reading/writing means (22) and able to process said information according to a predefined program, said processing unit (18) being connectable to external electronic devices.

2. Appliance according to claim 1, **characterized by** the fact that said storage means (21) are mounted on said second end (6) of the syringe device (2) and said reading/writing means (22) are positioned at said fastening seat (13) of the operating device (12), said reading/writing means (22) and said storage means (21) being arranged in such a way that, upon the coupling between said syringe device (2) and said operating device (12), they are directly connected the ones to the others.

3. Appliance according to one or more of the preceding claims, **characterized by** the fact that said storage means (21) include pre-stored information able to identify said syringe device (2).

4. Appliance according to one or more of the preceding claims, **characterized by** the fact that said control means (18) are programmable so as to automatically set the doses of fluid to take and dispense.

5. Appliance according to one or more of the preceding claims, **characterized by** the fact that it comprises a control interface (23) mounted on the operating device (12) and connected to the control means (18) so as to allow a user to set the quantity of liquid of the doses and, following the coupling between said syringe device (2) and said operating device (12), to allow said user to control the dispensing and/or the taking of fluid, and collect/transfer information from/onto said storage means (21).

6. Appliance according to one or more of the preceding claims, **characterized by** the fact that said piston (4) defines and separates, inside said syringe body (3), a containment chamber (8), which ends up at said first end (5), and a sliding chamber (9), meant for housing said thrust rod (15) and ending up in said second end (6), and by the fact that it comprises a covering element (24) arranged in said sliding chamber (9) and comprising an opening hole (25), so as to allow the temporary fastening of the rod (15) to said piston (4), said covering element (24) being configured in such a way as to cover the inner surface of the side wall/s of the sliding chamber (9), independently of the position of said piston (4) in said syringe body (3), so as to avoid that, upon the uncoupling between syringe device (2) and operating device (12), the air entering the syringe body (3) from said second end (6) can touch said inner surface.

7. Appliance according to the claim 6, **characterized by** the fact that said covering element (24) is longitudinal shaped and made of an elastic and resilient material, and by the fact that said piston (4) comprises an abutment side (26), facing said sliding chamber (9), which abuts a first longitudinal end (27) of said covering element (24), and by the fact that said second end (6) of the syringe body comprises an abutment transversal frame (28) defined around a through hole (29), said covering element (24) comprising a second longitudinal end (30) which abuts said abutment frame (28), and being fitted longitudinally pre-compressed in said sliding chamber (9) so that its first and second ends (27, 30) adhere to said abutment side (26) of the piston (4) and to said second abutment frame (28), respectively, independently of the position of the piston (4) itself, so as to define, between the outer side surface of the covering element (24) and said inner side surface of the syringe body (3), a sealed gap (31).

8. Appliance according to claim 6 or the claim 7, **characterized by** the fact that said covering element (24) is tubular, axial-symmetric and comprises one/some bellows-shaped side wall/s, so as to favor the variations of its longitudinal dimensions.

9. Appliance according to claim 7, **characterized by** the fact that said abutment side (26) of the piston (4) shapes an abutment seat (34) able to receive a portion of said covering element (24) which comprises said first end (27).

10. Appliance according to one or more of the preceding claims, **characterized by** the fact that said joining means are a Luer-lock connector.

## Patentansprüche

1. Vorrichtung (1) für das Überführen von biomedizinischen Fluiden zwischen Krankenhausbehältern (R, P), die geeignet sind für die Aufnahme, den Transport und die Abgabe von Dosen von biomedizinischem Fluid, umfassend:
- mindestens eine Spritzenvorrichtung (2), die ihrerseits einen hohlen Spritzenkörper (3) zur Aufnahme des biomedizinischen Fluids aufweist, und ebenfalls mindestens einen gleitenden Koiben (4), der abgedichtet in den Spritzenkörper (3) eingepasst ist, wobei letzterer ein erstes Ende (5) aufweist, das Verbindungsmittel für das in Verbindung setzen seiner Innenseite mit derjenigen eines Krankenhausbehälters (R, P) aufweist, und ein zweites offenes Ende (6) aufweist;
- mindestens eine greifbare Betätigungsvorrichtung (12), die einen Befestigungssitz (13) umfasst, der in einer entfernbaren Art und Weise an das zweite Ende (6) der Spritzenvorrichtung (2) gekoppelt werden kann, wobei die Betätigungsvorrichtung (12) eine Schubstange (15) umfasst, die geeignet ist, in Längsrichtung durch das zweite Ende (6) zu gleiten, nachfolgend auf die Koppelung zwischen der Spritzenvorrichtung (2) und der Betätigungsvorrichtung (12);
- temporäre Befestigungsmittel zum Verbinden der Stange (15) mit dem Kolben (4) in einer lösbaren Art und Weise, die ermöglichen können, dass der Betrieb der Stange (15) das Gleiten des Kolbens (4) in dem Spritzenkörper (3) bewirkt;
- Motormittel (17) zum Betätigen der Schubstange (15), aufgenommen in der Betätigungsvorrichtung (12);
- Steuermittel (18), aufgenommen in der Betätigungsvorrichtung (12) und mit den Motormitteln (17) verbunden, um so sowohl die volumetrische Dosierung der aus einem ersten Krankenhausbehälter (R) entnommenen Menge von Fluid als auch die volumetrische Dosierung der in einen zweiten Krankenhausbehälter (P) abgegebenen Menge von Fluid zu steuern;
wobei die Vorrichtung (1) **dadurch gekennzeichnet ist, dass**:
die Speichermittel (21) an der Spritzenvorrichtung (2) angebracht sind für das Speichern von Informationen in Bezug auf die Art des übertragenen Fluids, die Menge des entnommenen Fluids, die Menge des abgegebenen Fluids, wann jede Dosis entnommen und/oder abgegeben wurde und wie viele Dosen abgegeben/entnommen worden sind;
- die Betätigungsvorrichtung (12) Lese-/Schreibmittel (22) zum Lesen und Schreiben der Information von/auf die Speichermittel (21) umfasst; und dass
- die Steuermittel eine Verarbeitungseinheit (18) umfassen, welche die Lese-/Schreibmittel (22) steuern können und zum Verarbeiten der Informationen gemäß einem vorgegebenen Programm in der Lage sind, wobei die Verarbeitungseinheit (18) mit externen elektronischen Einrichtungen verbindbar ist.

2. Vorrichtung nach Anspruch 1, **dadurch gekennzeichnet, dass** die Speichermittel (21) an dem zweiten Ende (6) der Spritzenvorrichfiung (2) befestigt sind und die Lese-/Schreibmittel (22) an dem Befestigungssitz (13) der Betätigungsvorrichtung (12) positioniert sind, wobei die Lese-/Schreibmittel (22) und die Speichermittel (21) derart angeordnet sind, dass bei der Kopplung zwischen der Spritzenvorrichtung (2) und der Betätigungsvorrichtung (12) sie direkt mit der anderen verbunden werden.

3. Vorrichtung nach einem oder mehreren der vorhergehenden Ansprüche, **dadurch gekennzeichnet, dass** die Speichermittel (21) vorgespeicherte Informationen umfassen, weiche die Spritzenvorrichtung (2) identifizieren können.

4. Vorrichtung nach einem oder mehreren der vorhergehenden Ansprüche, **dadurch gekennzeichnet, dass** die Steuermittel (18) programmierbar sind, um automatisch die zu entnehmenden und die abzugebenden Dosen des Fluids einzusehen.

5. Vorrichtung nach einem oder mehreren der vorhergehenden Ansprüche, **dadurch gekennzeichnet, dass** sie eine Steuerschnittstelle (23) umfasst, die an der Betätigungseinrichtung (12) befestigt und mit den Steuermitteln (18) verbunden ist, um es so einem Benutzer zu ermöglichen, die Flüssigkeitsmenge der Dosen einzustellen und nachfolgend auf das Koppeln der Spritzenvorrichtung (2) und der Betätigungsvorrichtung (12) es dem Benutzer zu ermöglichen, das Abgeben und/oder das Entnehmen von Fluid zu steuern, und Informationen von/auf der/die Speichermittel (21) zu sammeln/zu übertragen.

6. Vorrichtung nach einem oder mehreren der vorhergehenden Ansprüche, **dadurch gekennzeichnet, dass** der Kolben (4) im Inneren des Spritzenkörpers (3) eine Aufnahmekammer (8), welche an dem ersten Ende (5) endet, und eine Gleitkammer (9) definiert und abtrennt, die zur Unterbringung der Schubstange (15) gedacht ist und in dem zweiten Ende (6) endet und dadurch, dass sie ein Abdeckelement (24) umfasst, das in der Gleitkammer (9) angeordnet ist, und ein Öffnungsloch (25) aufweist, um die temporäre Befestigung der Stange (15) an dem Kolben (4) zu ermöglichen, wobei das Abdeckelement (24) derart konfiguriert ist, dass es die Innenfläche von der Seitenwand/den Seitenwänden der Gleitkammer (9) abdeckt, unabhängig von der Position des Kolbens (4) in dem Spritzenkörper (3), um zu vermeiden, dass bei der Entkopplung zwischen der Spritzenvorrichtung (2) und der Betätigungsvorrichtung (12) Luft, die von dem zweiten Ende (6) in den Spritzenkörper (3) einbringt, die Innenfläche berühren kann.

7. Vorrichtung nach Anspruch 6, **dadurch gekennzeichnet, dass** das Abdeckelement (24) in Längsrichtung geformt und aus einem elastischen und federnden Material hergestellt ist, und dadurch, dass der Kolben (4) eine Anschlagseite (26) aufweist, welche der Gleitkammer (9) zugewandt ist, welche an ein erstes Längsende (27) des Abdeckelements (24) anstößt, und dadurch, dass das zweite Ende (6) des Spritzenkörpers einen Anschlagquerrahmen (28) aufweist, der rund um ein Durchgangsloch (29) definiert ist, wobei das Abdeckelement (24) ein zweites Längsende (30) ausweist, welches an den Anschlagrahmen (28) anstößt, und das in Längsrichtung vorkomprimiert in der Gleitkammer (9) angebracht ist, sodass seine ersten und zweiten Enden (27, 30) entsprechend an der Anschlagseite (26) des Kolbens (4) und dem zweiten Anschlagrahmen (28) anhaften, unabhängig von der Position des Kolbens (4) selbst, um zwischen der äußeren Seitenfläche des Abdeckelements (24) und der inneren Seitenfläche des Spritzenkörpers (3) einen abgedichteten Zwischenraum (31) zu definieren.

8. Vorrichtung nach Anspruch 6 oder 7, **dadurch gekennzeichnet, dass** das Abdeckelement (24) rohrförmig, axial-symmetrisch ist und eine/einige baigförmige Seitenwand/Seitenwände umfasst, um so die Variationen seiner Längsabmessungen zu begünstigen.

9. Vorrichtung nach Anspruch 7, **dadurch gekennzeichnet, dass** die Anschlagseite (26) des Kolbens (4) einen Anschlagsitz (34) formt, der in der Lage ist, einen Abschnitt des Abdeckelements (24) auszunehmen, der das erste Ende (27) umfasst.

10. Vorrichtung nach einem oder mehreren der vorhergehenden Ansprüche, **dadurch gekennzeichnet, dass** die Verbindungsmittel ein Luer-Lock-Anschluss sind.

## Revendications

1. Appareil (1) de transfert de fluides biomédicaux entre réceptacles d'hôpital (R, P), apte à prélever, transporter et distribuer des doses de fluide biomédical, comprenant :
- au moins un dispositif de seringue (2) comprenant à son tour un corps de seringue creux (3) destiné à contenir ledit fluide biomédical, et comprenant en outre au moins un piston coulissant (4) monté étanche dans ledit corps de seringue (3), ce dernier comprenant une première extrémité (5), possédant des moyens de raccordement pour mettre en communication son intérieur avec celui d'un récipient hospitalier (R, P), et une seconde extrémité ouverte (6);
- au moins un dispositif de commande préhensible (12), comprenant un siège d'attache (13) qui peut être couplé de manière amovible à ladite seconde extrémité (6) du dispositif de seringue (2), le dispositif de commande (12) comprenant une tige de poussée (15) apte à coulisser longitudinalement à travers ladite second extrémité (6), suite au couplage entre ledit dispositif de seringue (2) et ledit dispositif de commande (12) ;
- des moyens de fixation temporaire pour coupler ladite tige (15) audit piston (4) de manière libérable, capable de permettre que la manoeuvre de ladite tige (15) entraîne le coulissement dudit piston (4) dans ledit corps de seringue (3) ;
- des moyens de motorisation (17) pour entraîner ladite tige de poussée (15), compris dans ledit dispositif de commande (12) ;
- des moyens de commande (18), compris dans ledit dispositif de commande (12), et reliés auxdits moyens de motorisation (17) afin de commander le dosage volumétrique de la quantité de fluide prélevée d'un premier réceptacle d'hôpital (R) et le dosage volumétrique de la quantité de fluide distribuée dans un second réceptacle d'hôpital (P) ;
ledit appareil (1) étant **caractérisé en ce que** :
- des moyens de stockage (21) sont montés sur ledit dispositif de seringue (2) pour stocker des informations par rapport au type de fluide transféré, à la quantité de fluide prélevée, à la quantité de fluide distribuée, au moment de prélèvement et/ou de distribution de chaque dose et au nombre de doses distribuées/prélevées ;
- ledit dispositif de commande (12) comprend des moyens de lecture/écriture (22) pour la lecture et l'écriture desdites informations depuis/vers lesdits moyens de stockage (21) ; et **en ce que**
- lesdits moyens de commande comprennent une unité de traitement (18) apte à commander lesdits moyens de lecture/écriture (22) et apte à traiter lesdites informations selon un programme prédéfini, ladite unité de traitement (18) pouvant être reliée à des dispositifs électroniques externes.

2. Appareil selon la revendication 1, **caractérisé en ce que** lesdits moyens de stockage (21) sont montés sur ladite seconde extrémité (6) du dispositif de seringue (2) et lesdits moyens de lecture/écriture (22) sont positionnés au niveau dudit siège d'attache (13) du dispositif de commande (12), lesdits moyens de lecture/écriture (22) et lesdits moyens de stockage (21) étant agencés de telle manière que, lors du couplage entre ledit dispositif de seringue (2) et ledit dispositif de commande (12), ils sont directement reliés les uns aux autres.

3. Appareil selon l'une ou plusieurs des revendications précédentes, **caractérisé en ce que** lesdits moyens de stockage (21) comprennent des informations pré-stockées aptes à identifier ledit dispositif de seringue (2).

4. Appareil selon l'une ou plusieurs des revendications précédentes, **caractérisé en ce que** lesdits moyens de commande (18) sont programmables afin de régler automatiquement les doses de fluide à prélever et à distribuer.

5. Appareil selon l'une ou plusieurs des revendications précédentes, **caractérisé en ce qu'**il comprend une interface de commande (23) montée sur le dispositif de commande (12) et reliée aux moyens de commande (18) afin de permettre à un utilisateur de régler la quantité de liquide des doses et, suite au couplage entre ledit dispositif de seringue (2) et ledit dispositif de commande (12), de permettre audit utilisateur de commander la distribution et/ou le prélèvement de fluide, et de collecter/transférer des informations depuis/vers lesdits moyens de stockage (21).

6. Appareil selon l'une ou plusieurs des revendications précédentes, **caractérisé en ce que** ledit piston (4) définit et sépare, à l'intérieur dudit corps de seringue (3), une chambre de confinement (8), qui aboutit à ladite première extrémité (5), et une chambre de coulissement (9), destinée à loger ladite tige de poussée (15) et aboutissant dans ladite seconde extrémité (6), et **en ce qu'**il comprend un élément couvrant (24) agencé dans ladite chambre de coulissement (9) et comprenant un trou d'ouverture (25), afin de permettre la fixation temporaire de la tige (15) audit piston (4), ledit élément couvrant (24) étant agencé de manière à couvrir la surface intérieure de la/des paroi(s) latérale(s) de la chambre de coulissement (9), indépendamment de la position dudit piston (4) dans ledit corps de seringue (3), afin d'éviter que, lors du découplage entre le dispositif de seringue (2) et le dispositif de commande (12), l'air entrant dans le corps de seringue (3) depuis ladite seconde extrémité (6) puisse toucher ladite surface intérieure.

7. Appareil selon la revendication 6, **caractérisé en ce que** ledit élément couvrant (24) est de forme longitudinale et réalisé en un matériau élastique et résilient, et **en ce que** ledit piston (4) comprend une face de butée (26), en regard de ladite chambre de coulissement (9), qui vient en butée contre une première extrémité longitudinale (27) dudit élément couvrant (24), et **en ce que** ladite seconde extrémité (6) du corps de seringue comprend un cadre de butée transversal (28) défini autour d'un trou traversant (29), ledit élément couvrant (24) comprenant une seconde extrémité longitudinale (30) qui vient en butée contre ledit cadre de butée (28), et étant monté longitudinalement précomprimé dans ladite chambre de coulissement (9) de sorte que ses première et seconde extrémités (27, 30) adhèrent à ladite face de butée (26) du piston (4) et audit second cadre de butée (28), respectivement, indépendamment de la position du piston (4) lui-même, afin de définir, entre la surface latérale extérieure de l'élément couvrant (24) et ladite surface latérale intérieure du corps de seringue (3), un interstice étanche (31).

8. Appareil selon l a revendication 6 ou la revendication 7, **caractérisé en ce que** ledit élément couvrant (24) est tubulaire, axialement symétrique et comprend une/des paroi(s) latérale(s) en forme de soufflet, afin de privilégier les variations de ses dimensions longitudinales.

9. Appareil selon la revendication 7, **caractérisé en ce que** ladite face de butée (26) du piston (4) forme un siège de butée (34) apte à recevoir une portion dudit élément couvrant (24) qui comprend ladite première extrémité (27).

10. Appareil selon l'une ou plusieurs des revendications précédentes, **caractérisé en ce que** lesdits moyens de raccordement sont un connecteur Luer-Lock.
